# EUROPÄISCHE PATENTSCHRIFT

(11) **EP 3 173 784 B1**
(45) Veröffentlichungstag und Bekanntmachung des Hinweises auf die Patenterteilung: **18.11.2020**
(21) Anmeldenummer: 16020463.2
(22) Anmeldetag: 23.11.2016
(51) Int. Cl.: G01N 33/00

(54) **GASMESSANORDNUNG MIT PRÜFGASERZEUGUNGSEINHEIT**
GAS MEASURING ARRANGEMENT WITH TEST GAS GENERATION UNIT
COMPTEUR DE GAZ AVEC UNITÉ DE PRODUCTION DE GAZ DE CONTROLE

(30) Priorität: 24.11.2015 DE 102015015164
(43) Veröffentlichungstag der Anmeldung: 31.05.2017
(73) Patentinhaber: LAMTEC Meß- und Regeltechnik für Feuerungen GmbH, 69190 Walldorf (DE)
(72) Erfinder: Weber, Harald, 76684 Östringen (DE); Hammer, Frank, 75015 Bretten (DE)

(56) Entgegenhaltungen:
- DE-A1- 4 433 607
- DE-A1-102005 028 246
- DE-A1-102014 108 109
- DE-U1- 29 521 224
- US-A1- 2007 178 015

## Beschreibung

Die Erfindung geht aus von einer Gasmessanordnung, welche eine Gassensorvorrichtung umfasst, die ein Gassensorelement mit einer Messzone enthält, und ein Messgerät, das eine Messsignalverarbeitungs- und Bereitstellungseinheit enthält, gemäß dem Oberbegriff des Anspruchs 1.

Eine gattungsgemäße Gassensoranordnung dient der Bestimmung der Konzentration eines Stoffes oder eines Stoffgemisches in einem Gas und ist prinzipiell bekannt. Insbesondere geht die Erfindung dabei aus von einer Gasmessanordnung, um in einem verfahrenstechnischen Prozess die Bestimmung einer Komponente eines Gasgemisches vorzunehmen, sei es quantitativ oder qualitativ, und die Information über das Vorhandensein und ggf. die Konzentration der zu bestimmenden Gaskonzentration einem Prozessüberwachungs- oder Regelsystem zur weiteren Verwendung zur Verfügung zu stellen.

Typisches Beispiel ist die Verbrennungsüberwachung oder -regelung in einer Feuerungsanlage, in der Brennstoffe wie Öl oder Gas oder Holz oder Kohle verbrannt werden. Hier wird mittels einer Gassensoranordnung die Zusammensetzung des Verbrennungsabgases im Abgasstrom, sei es noch im Verbrennungsraum oder im Abgasrohr, erfasst und überwacht. Aus den Signalen der Gassensoranordnung erhält die Brennerregelungsvorrichtung oder das Feuerungsmanagementsystem Informationen darüber, ob die Verbrennung vollständig, also mit hohem Wirkungsgrad und niedrigem Schadstoffausstoß, abläuft oder nicht. Typischerweise werden hierzu Gassensorelemente eingesetzt, die direkt im Abgasstrom den im Abgas noch vorhandenen Restsauerstoffgehalt und/oder den Gehalt an unverbrannten Brennstoffbestandteilen erfassen. Unverbrannte Brennstoffbestandteile, aus deren Vorhandensein im Abgas sich Rückschlüsse auf die Qualität der Verbrennung schließen lassen, sind beispielsweise Kohlenmonoxid CO, Wasserstoff H₂, Kohlenwasserstoffe CₙHₘ. Die Summe der unverbrannten Bestandteile wird nachfolgend mit COe bezeichnet.

Ein dabei zum Einsatz kommendes Gassensorelement hat eine Anordnung, in der mittels eines sensitiven Materials eine chemische oder physikalische Größe, beispielsweise die Konzentration eines Stoffes oder Stoffgemisches, möglichst selektiv und reversibel in ein elektrisches Signal umgewandelt wird, welches von der Konzentration des zu bestimmenden Stoffes oder Stoffgemisches abhängig ist. Dort, wo die genannte Umwandlung der chemischen oder physikalischen Größe in ein elektrisches Signal erfolgt, befindet sich die Messzone des Gassensorelementes. In dem sensitiven Material des Sensors wird dabei durch den zu bestimmenden Stoff eine Änderung einer chemischen, elektrochemischen oder physikalischen Eigenschaft hervorgerufen, welche durch einen Signalüberträger zur Änderung einer elektrischen Messgröße führt. Üblicherweise wird eine Messelektronik verwendet, um das elektrische Signal der Messgröße in ein Signal zur rechnergestützten Aufnahme und Auswertung aufzubereiten. Dazu umfasst die Messelektronik oft auch einen Mikroprozessor.

In der DE 101 53 643 A1 ist ein mit einer Heizvorrichtung beheizter keramischer Festelektrolytsensor zur Erfassung einer Gaskonzentration gezeigt, wobei der Sensor mindestens zwei an einer ionenleitenden Keramik, die mit einer Heizvorrichtung beheizbar ist, angeordnete Sensorelektroden umfasst, deren Sensorspannung in einer den Sensorelektroden nachgeschalteten Auswerteschaltung ausgewertet wird, und eine Feuerungsanlage, die einen mit festem, flüssigen oder gasförmigem Brennstoff befeuerten Brenner, ein Luft- und/oder Abgasgebläse, eine Regeleinrichtung und wenigstens einen mit einer Heizvorrichtung beheizten Sensor zur Erfassung einer Gaskonzentration umfasst, dessen Sensorspannung der Regeleinrichtung zur Verfügung gestellt wird.

Keramische Festelektrolytsensoren eignen sich aufgrund ihrer hohen Betriebstemperatur und ihrer Robustheit gut für den in-situ-Einsatz, direkt im Abgasstrom einer Feuerungsanlage. Dazu wird das Gassensorelement in einer Einbauarmatur untergebracht, hier auch als Gassensorvorrichtung bezeichnet, und durch eine Öffnung in der Abgasrohrwand in dem Abgasstrom platziert. Es entfällt hierbei die Notwendigkeit einer Messgasentnahme und Messgasaufbereitung, wie sie bei anderen ex-situ Gasanalysatoren erforderlich ist. Das erlaubt eine kostengünstige, direkte in-situ-Erfassung der die Verbrennungsqualität indizierenden Gasbestandteile und eine verzögerungsfreie Echtzeitmessungen, was für eine präzise und schnelle Brennerregelung von Vorteil ist.

Die Messelektronik zur Erfassung und Aufbereitung der Sensorsignale befindet sich in einem Messgerät. Dieses kann sich im Einsatz auch getrennt von der Einbauarmatur mit dem Gassensorelement, räumlich getrennt von der Gassensorvorrichtung, befinden. Eine Signalvorverarbeitung, beispielsweise eine Vorverstärkung, kann sich auch schon in der Gassensorvorrichtung, nahe dem Gassensorelement, befinden. Das elektrische Signal der Messgröße wird von dem Gassensorelement beziehungsweise von der Gassensorvorrichtung zu dem Messgerät mittels einer Signalleitung, einer Leiteranordnung, hier allgemein als Signalübertragungsmittel bezeichnet, übertragen. Es kann auch eine an sich bekannte kabellose Signalübertragung, beispielsweise per Funk, eingesetzt werden. Ein solches Messgerät wird auch allgemein als Transmitter bezeichnet, da es die Messsignale in aufbereiteter Form an z.B. eine Brennerregelung weitergeben kann.

Der Messwert wird dann einer Regeleinrichtung, wie sie beispielsweise in der EP 0 697 564 A1 offenbart ist, zugeführt. Die Regeleinrichtung kann in dem Messgerät enthalten sein.

Es ist auch denkbar, Brennerregelung und Transmitter in einem Gerät zu vereinigen. Auch ein solches Kombinationsgerät soll hier unter dem Begriff "Messgerät" mit verstanden werden.

Die Regeleinrichtung regelt unter anderem die Menge an Brennstoff und Luft, die dem Brenner zugeführt werden, unter Verwendung des Sensorsignals beispielsweise derart, dass die Verbrennung mit einem definierten, beispielsweise einem möglichst geringen, Luftüberschuss, aber dennoch vollständig, d.h., ohne dass in nennenswertem Maße unverbrannte Gasbestandteile im Abgas auftreten, erfolgt. Selbstverständlich muss eine Brennstoff-Luft-Regelung nicht auf das minimal mögliche Brennstoff-Luft-Verhältnis, auch Lambda-Zahl genannt, eingestellt sein, sondern es kann auf jede beliebige Lambdazahl, z.B. auch auf einen höheren Luftüberschuss, also ein höheres Lambda als das minimal mögliche Lambda, geregelt werden.

Bei Sensoren wie den Festkörper-Elektrolyt-Sensoren kann es im Laufe der Betriebszeit zu alterungsbedingten Veränderungen des Sensorverhaltens und auch zu Ausfällen kommen. Ein Festkörper-Elektrolyt COe-Sensor würde beispielsweise in einem fehlerhaften Zustand trotz vorhandenem unverbrannten Gasbestandteilen im Abgas eine nur geringe Sensorspannung abgeben, woraus die Brennerregelung dann den Schluss ziehen könnte, die Menge an zugeführter Verbrennungsluft noch weiter zu reduzieren, wodurch die Verbrennung schließlich immer weiter in den Bereich der Unvollständigkeit hineingefahren würde. Ein Festkörper-Elektrolyt O₂-Sensor würde in einem fehlerhaften Zustand beispielsweise nicht den tatsächlichen sondern einen davon abweichenden, z.B. verringerten und damit falschen O₂-Anteil liefern, woraus die Brennerregelung dann den Schluss ziehen könnte, die Menge an zugeführter Verbrennungsluft zu erhöhen, wodurch die Verbrennung schließlich immer weiter in den Bereich des Luftüberschusses mit entsprechendem Wirkungsgradverlust hineinfahren würde. Ein fehlerhafter Zustand des Sensors kann unterschiedliche Ursachen haben. Beispielsweise könnten die Mess-Elektroden altern oder sich ablösen oder durch Sensorgifte verändert werden, oder die Heizwendel könnte altern, wodurch der Sensor trotz korrekt anliegender Heizleistung nicht seine erforderliche Betriebstemperatur erreicht, oder es könnte ein Kabelbruch vorliegen, oder es könnten sich Veränderungen in den Signalleitungen oder den Schweiß- und Lötstellen eingestellt haben. Eine weitere Quelle möglicher Sensorbeeinträchtigungen sind Ablagerungen, etwa aus dem Abgas des Brenners oder durch abgedampftes Isolationsmaterial aus dem Bereich der Sensorbefestigung. Solche Ablagerungen stören die Wechselwirkungen der Gasmoleküle mit den Elektroden und dem Festkörperelektrolyten an der Dreiphasengrenze, so dass ein fehlerhaftes Sensorsignal erzeugt wird, auch wenn ansonsten die Elektroden und die Heizung in Ordnung sind und keine weiteren Alterungseffekte zeigen.

Die Funktionstüchtigkeit des Sensors ist im Zusammenspiel mit der Brennerregelung eine sicherheitsrelevante Funktion des Systems Feuerungsanlage. Wenn der Sensor fehlerhafte Informationen liefert, kann die Brennersteuerung die Feuerungsanlage in einen fehlerhaften und möglicherweise sogar gefährlichen Betriebszustand fahren.

Es ist bekannt, das Gassensorelement mittels eines Plausibilitätschecks im eingebauten Zustand zu überprüfen. Dazu wird durch die Brennerregelung das Brennstoff-Luft-Verhältnis kurzzeitig so verändert, dass es zu einer Reaktion des Gassensorelementes kommen muss. Weicht dann die Sensorreaktion von der Erwartung ab, wird z.B. keine Reaktion des Sensors erfasst oder eine zu schwache Reaktion, so ist das ein Hinweis darauf, dass mit dem Gassensor etwas nicht in Ordnung sein könnte. Diese Art der Funktionsüberprüfung kann jedoch nicht unabhängig von der Einbauumgebung bzw. nicht ohne die Feuerungsanlage und den dort provozierten Aktionen und damit Reaktionen des Gassensorelementes durchgeführt werden.

Zur Erfüllung einschlägiger Normen der funktionalen Sicherheit, beispielsweise der internationalen Normung gemäß IEC 61508/IEC61511 und dem Erreichen von darin beschriebenen sogenannten Sicherheits-Integritäts-Levels (SIL), beispielsweise SIL 2 oder SIL3, muss eine Funktionsüberprüfung der Gasmessanordnung bzw. der Brennerregelung im Zusammenspiel mit dem Gassensor jedoch jederzeit und unabhängig von der Feuerungsanlage möglich sein.

Regelmäßige Funktionsüberprüfungen des Sensors sind allerdings aufwändig, da der Sensor hierzu z.B. ausgebaut, untersucht und auf Funktion getestet sowie gegebenenfalls mittels Prüfanlage mit Prüfgas hinsichtlich seines Messverhaltens überprüft werden müsste. Dadurch wird nicht nur der Brennerbetrieb beeinträchtigt. Dies bedeutet auch einen hohen Aufwand an Arbeitszeit und Materialkosten.

Die US 2007/0178015 A1 zeigt eine Kalibriergaszuführungsvorrichtung zum Zuführen eines wahlweise befeuchteten Kalibriergases zu einer Messsonde, wobei die Vorrichtung eine Zufuhrleitung, die einen Leitungseinlass zum Aufnehmen eines Trägergasstroms und einen Leitungsauslass zum Zuführen eines Kalibriergasstroms aufweist, umfasste, sowie eine Massendurchflusssteuerungseinheit, die angrenzend an den Leitungseinlass der Zufuhrleitung angeordnet ist, und die den Durchfluss des Trägergases steuert. Weiter ist vorhanden eine erste Injektionseinheit, die eine erste Ansaugöffnung in Fluidkommunikation mit einem ersten Reservoir und einen ersten Auslass in Fluidkommunikation mit der Zufuhrleitung aufweist, wobei das erste Reservoir eine ionische Quecksilberlösung in flüssiger Form enthalt, sowie eine zweite Injektionseinheit, die eine zweite Ansaugöffnung in Fluidkommunikation mit einem zweiten Reservoir und eine zweite Auslassöffnung in Fluidkommunkation mit der Zufuhrleitung aufweist, wobei das zweite Reservoir Wasser als ein Befeuchtungsmittel in flüssiger Form enthält. Weiterhin gibt es mindestens einen Verdampfer stromabwärts des ersten und des zweiten Auslasses und stromaufwärts des Leitungsauslasses, der die ionische Quecksilberlösung und das Wasser in Dampfform umwandelt und das Kalibriergas, welches das Trägergas umfasst, den ionischen Quecksilberlösungsdampf und Wasserdampf dem Leitungsauslass zuführt.

Die DE 10 2005 028 246 A1 zeigt eine kompakte Gassensoranordnung, welche ohne den Einsatz mechanischer Bauelemente mit einem Prüfgas beaufschlagt werden kann und deren Messbereitschaft während der Kalibrierung nur kurzzeitig unterbrochen ist, bestehend aus der Kombination eines elektrochemischen Gassensors und eines elektrochemischen Gasgenerators mit einem Gaskanal vom Gasgenerator zur Messelektrode des Gassensors, wobei das Messgas aus der Umgebung des Gassensors freien Zugang zur Messelektrode hat

Es ist daher die der vorliegenden Erfindung zugrundeliegende Aufgabe, eine Gasmessanordnung zu schaffen, die eine von der Prozessanlage unabhängige Funktionsüberprüfung des Messgerätes mit angeschlossenem Gassensorelement auf einfache und unkomplizierte Weise sowie darüber hinaus bei vorteilhafter Ausgestaltung eine Kalibrierung erlaubt.

Die Aufgabe wird gelöst durch eine Gasmessanordnung mit den kennzeichnenden Merkmalen des Anspruchs 1.

Erfindungsgemäß also enthält das Messgerat zusätzlich eine Prüfgasbereitstellungseinheit zur Bildung eines Prüfgases vorbestimmbarer und/oder veränderbarer Prüfgaszusammensetzung, und die Gasmessanordnung umfasst ein Gaszuführungsmittel, um das Prüfgas von der Prüfgasbereitstellungseinheit in dem Messgerät dem Gassensorelement zuzuführen.

Die erfindungsgemäße Gasmessanordnung erlaubt es, eine Funktionsüberprüfung des Gassensorelementes jederzeit auch automatisch und unabhängig von der Prozessanlage, in der die Gassensorvorrichtung mit dem Gassensorelement eingebaut ist, vorzunehmen.

In vorteilhafter Ausführung kann aus der reinen Funktionsprüfung ein Abgleich zur Kompensation von Alterung oder Drift bzw. eine Kalibrierung entstehen, die die Gasmessanordnung als Messmittel qualifiziert.

An das Gassensorelement selbst sind keine besonderen Anforderungen zu stellen. Es kann jedes üblicherweise verwendete Gassensorelement verwendet werden, beispielsweise ein planares Zirkondioxid-basiertes Gassensorelement, oder auch ein bekanntes Gassensorelement mit einem einseitig geschlossenen Zirkondioxidröhrchen, das eine außenliegende Messgasseite und eine innenliegende Referenzgasseite hat.

Das Prüfgas wird an die Einbauarmatur, die Gassensorvorrichtung, herangeführt. Innerhalb der Einbauarmatur umspült das Prüfgas das Gassensorelement und wird danach an die Umgebung bzw. in den Prozess oder den Abgaskanal entlassen, dazu ist nur eine sehr geringe Modifikation an der Einbauarmatur vorzunehmen, es ist nur ein Anschlussmittel zum Anschluss z.B. eines Prüfgasschlauches, dem Gaszuführungsmittel, vorzusehen. Es sind keine externen Prüfgasflaschen erforderlich, die Bereitstellung des Prüfgases erfolgt innerhalb des Messgerätes. Die Funktionsüberprüfung beziehungsweise eine Kalibrierung kann unabhängig von der Einbaubedingung des Gassensors erfolgen, also sowohl wenn der Gassensor in einem Abgasrohr oder in einer Prozesskammereingebaut ist, als auch wenn er sich außerhalb befindet.

Gemäß einer vorteilhaften Ausführungsform der Erfindung hat die Prüfgasbereitstellungseinheit eine Erzeugungseinheit zur Erzeugung eines oder mehrerer Teilströme mit Gasbestandteilen des Prüfgases, eine Trägergasbereitstellungseinheit zur Bereitstellung eines Trägergas-Teilstroms und eine Prüfgaszusammensetzungseinheit zur Mischung des Trägergas-Teilstroms mit dem oder den in der Erzeugungseinheit bereitgestellten Teilströmen der Gasbestandteile.

Gemäß einer vorteilhaften Ausführungsform können die Prüfgase auch direkt und ohne Mischung ins Trägergas an das Gassensorelement herangeführt werden.

Eine Funktionseinheit umfassend die Kombination aus Erzeugungseinheit, Trägergasbereitstellungseinheit und Prüfgaszusammensetzungseinheit kann in einem Ausführungsbeispiel eine mit Wasser und beispielsweise Alkohol gefüllte Kartusche umfassen, über die ein Prüf-Luftstrom in geeigneter Weise geführt wird, derart, dass der Prüf-Luftstrom mit Alkoholdämpfen angereichert wird. Der mit Alkoholdämpfen angereicherte Prüf-Luftstrom ist dann das im Messgerät erzeugte Prüfgas.

Die Luft für den Prüf-Luftstrom kann Druckluft sein. Gemäß einer vorteilhaften Ausführungsform der Erfindung hat die Trägergasbereitstellungseinheit dazu einen Anschluss an eine Druckluftversorgung von außerhalb des Messgerätes her, und die Trägergasbereitstellungseinheit hat eine Vorrichtung zum Erzeugen eines Druckluft-Teilstromes mit vorgebbarer Flussrate.

Die Luft für den Prüf-Luftstrom kann auch aus der Umgebungsluft genommen werden. Gemäß einer vorteilhaften Ausführungsform der Erfindung hat die Trägergasbereitstellungseinheit dazu einen Kompressor oder eine Pumpe oder einen Ventilator oder ein Gebläse zur Erzeugung eines Druckluft-Teilstromes aus der Umgebungsluft.

In einer vorteilhaften Ausführungsform kann, wenn in dem Abgasrohr oder in dem Prozess, in den die Gassensorvorrichtung eingebaut ist, ein Unterdruck gegenüber der Umgebung herrscht, dies kann in einem Kamin beispielsweise der Kaminzug sein, dieser Unterdruck bzw. Kaminzug genutzt werden, um den benötigten Luft-teilstrom aus der Umgebungsluft abzuziehen. In diesem Fall wird dann keine Pumpe oder kein Ventilator benötigt.

Eine weitere, vorteilhafte Möglichkeit zur Erzeugung eines Prüfgases innerhalb des Messgerätes besteht in der Verwendung einer Elektrolysezelle, beispielsweise einer Polymer-Elektroden-Elektrolysezelle, kurz PEM-Elektrolysezelle, an die eine geeignete Elektrolysespannung zur Erzeugung von H₂ und O₂ angelegt wird. Gemäß einer vorteilhaften Ausführungsform der Erfindung ist die Erzeugungseinheit dabei mit einer Elektrolysezelle versehen zur Erzeugung eines Sauerstoff-Teilstroms als Teilstrom eines ersten Gasbestandteils des Prüfgases und zur Erzeugung eines Wasserstoff-Teilstromes als Teilstrom eines zweiten Gasbestandteiles des Prüfgases. Die Menge an H₂ und O₂, die in der Elektrolysezelle erzeugt wird, ist von der Höhe der Elektrolysespannung abhängig.

Daher erlaubt die Verwendung einer Elektrolysezelle durch Anlegen unterschiedlicher Elektrolysespannungen, bzw. die Heranziehung des Elektrolysestromes als Maß für den Stoffumsatz auch die definierte Erzeugung unterschiedlicher H₂ und O₂ - Mengen. Dies ermöglicht die Erzeugung von Prüfgasströmen mit unterschiedlich vorgebbaren H₂- und O₂ - Anteilen, mit denen der Gassensor nicht nur auf Funktion getestet, sondern auch an mehreren Stützstellen kalibriert werden kann. Dafür müssen keine Testgasflaschen mit unterschiedlicher Prüfgaskonzentration mitgeführt werden, sondern mittels der Elektrolysezelle können unterschiedliche Prüfgaskonzentrationen mittels Vorgeben unterschiedlicher Elektrolysespannungen in dem Messgerät selbst erzeugt werden.

Gemäß einer vorteilhaften Ausführungsform ist die Erzeugungseinheit mit wenigstens einem ersten und einem zweiten Behälter, auch als Kartusche zu bezeichnen, versehen, wobei in dem wenigsten ersten Behälter auf chemischem Wege O₂ erzeugt wird und wobei in dem wenigsten zweiten Behälter auf chemischem Wege H₂ erzeugt wird. Gemäß einer vorteilhaften Ausführungsform befindet sich beispielsweise in dem wenigstens ersten Behälter zur O₂-Erzeugung eine erste Lösung, beispielsweise eine ölige Flüssigkeit, in der Sauerstoff chemisch gebunden ist, und ein Katalysator, wobei durch den Katalysator katalytisch unterstützt in der ersten Lösung eine chemische Reaktion unter unter Bildung von O₂ abläuft, und dass in dem wenigstens zweiten Behälter zur H₂-Erzeugung sich eine zweite Lösung, beispielsweise eine andere ölige Flüssigkeit mit einer von der ersten Lösung unterschiedlicher Zusammensetzung, befindet, in der Wasserstoff chemisch gebunden ist, und ein anderer Katalysator, wobei durch den Katalysator katalytisch unterstützt in der zweiten Lösung eine chemische Reaktion unter Bildung von H₂ abläuft. Die erste und zweite Lösung und die jeweiligen Katalysatoren werden in ihrer Zusammensetzung und chemischen Beschaffenheit so ausgewählt, dass die jeweilige chemische Reaktion unter Bildung von Sauerstoff bzw. Wasserstoff über einen längeren Zeitraum hinweg, beispielsweise über ein halbes Jahr, mit einer solchen gleichmäßigen Umsetzungsrate abläuft, dass während dieser ganzen Zeit ausreichend Sauerstoff bzw. Wasserstoff entsteht. Nach Ablauf dieser Zeit ist der Behälter erschöpft und kann einfach gegen einen neuen ausgetauscht werden.

In dieser Ausführungsform kann die Lösung und der Katalysator auch in Abwandlung so gewählt und aufeinander abgestimmt sein, dass ein anderes oxidierbares Gas, anders als Wasserstoff, entsteht, beispielsweise könnte ein Alkohol als Reaktionsprodukt entstehen oder CO. Auf diese Weise kann dann der Prüfgasstrom mit diesem anderen oxidierbaren Gasbestandteil gebildet werden.

Gemäß einer vorteilhaften Ausführungsform der Erfindung hat die Prüfgaszusammensetzungseinheit eine Mischeinheit zur Mischung des Trägergas-Teilstroms mit dem oder den Teilströmen der Gasbestandteile. Es können damit in sehr variabler Weise Prüfgasströme mit unterschiedlicher Zusammensetzung in dem Messgerät erzeugt werden. Ein Prüfgasstrom einer ersten Art kann zum Beispiel ein Luft/H₂/O₂ -Gemisch sein. Ein Prüfgasstrom einer zweiten Art kann ein Luft/H₂-Gemisch sein. Ein Prüfgasstrom einer dritten Art kann ein Luft/O₂-Gemisch sein. Ein Prüfgasstrom einer vierten Art kann reine Luft sein. Ein Prüfgasstrom einer fünften Art kann reiner H₂ sein. Ein Prüfgasstrom einer sechsten Art kann reiner O₂ sein. Wenn in diesem Zusammenhang von reinen Gasen gesprochen wird, so ist damit gemeint, dass produktionsbedingte leichte Verunreinigungen im Rahmen der vom Hersteller angegebenen Gasreinheitsklasse gegebenenfalls noch vorhanden sein können. So ist es durchaus üblich, dass in einem reinen O₂ - Prüfgas noch einige ppm H₂ vorhanden sein können, oder in reinem H₂ - Prüfgas noch einige ppm O₂. Diese Teilströme können dabei direkt erzeugt und kontinuierlich oder gespeichert und als Messgasstoß über das Gaszuführungsmittel an das Sensorelement herangeführt werden.

Wenn ein Sensor mit einer Messgasseite und einer Referenzgasseite verwendet wird, so kann das Gaszuführungsmittel sowohl an die Messgasseite und/oder an die Referenzgasseite herangeführt werden.

Zur definierten Einstellung und Mischung der Teilvolumenströme können in der Mischeinheit und/oder in den Trägergas-, Testgas- und Prüfgasleitungen sogenannte kritische Düsen eingesetzt werden. Eine kritische Düse ist im Prinzip bekannt. Sie zeichnet sich dadurch aus, dass, solange ein kritisches Druckverhältnis, ein kritischer Druckabfall über der Düse aufrechterhalten oder überschritten ist, der Volumenstrom durch die kritische Düse hindurch konstant und weitgehend unabhängig von Druckschwankungen ist. Der kritische Druckabfall ist von geometrischen Düsenparametern bestimmt.

In einer vorteilhaften Ausführungsform kann eine kritische Düse, die den Volumenstrom des Prüfgases einstellt, in oder nahe der Gassensorvorrichtung mit dem beheizten Gassensorelement angebracht sein. Die höhere und konstant geregelte Temperatur der Gassensorvorrichtung würde dann auch die kritische Düse auf einer gegenüber der Umgebungstemperatur erhöhten höheren und konstanteren Temperatur belassen, wodurch sich Temperaturschwankungen der Umgebungstemperatur und/oder der Prüfgastemperatur weniger stark auf die kritische Düse auswirken würden und sich keine Kondensattropfen bilden könnten, was ansonsten bei Bildung von Kondensattropfen die Gefahr einer Düsenverstopfung mit sich brächte. Dadurch lässt sich der Einfluss von Dichteschwankungen des Prüfgases, die durch möglicherweise im Prüfgas oder der Umgebung der Prüfgasleitungen auftretende Temperaturschwankungen bedingt sind, auf den Volumenstrom des Prüfgases verringern.

Die Aufrechterhaltung des kritischen Druckverhältnisses an den kritischen Düsen kann durch Beaufschlagen mit Überdruck oder durch die Erzeugung von Unterdruck erfolgen.

In einer vorteilhaften Ausführungsform kann, wenn in dem Abgasrohr oder in dem Prozess, in den die Gassensorvorrichtung eingebaut ist, ein Unterdruck gegenüber der Umgebung herrscht, dies kann in einem Kamin beispielsweise der Kaminzug sein, dieser Unterdruck bzw. Kaminzug zur Aufrechterhaltung des kritischen Druckverhältnisses an den kritischen Düsen genutzt werden. Es könnte auch ein Überdruck, der sich durch die Gasproduktion der Elektrolysezelle einstellt, zur Aufrechterhaltung des kritischen Druckverhältnisses an den kritischen Düsen genutzt werden.

Die Prüfgaszusammensetzungseinheit kann in einer vorteilhaften Ausführungsform Pumpen auf der Saug- oder Zugseite der Testgas-, Trägergas- und/oder Prüfgasleitungen einsetzen.

In einer weiteren vorteilhaften Ausführungsform kann die Prüfgaszusammensetzungseinheit auch mit Massenflussreglern, die nach einem thermischen und/oder elektropneumatischen oder anderen bekannten Prinzip arbeiten, zur definierten Einstellung der jeweiligen Volumenströme ausgestattet sein.

Anhand der Zeichnungen, in der drei Ausführungsbeispiele der Erfindung dargestellt sind, sollen die Erfindung sowie weitere vorteilhafte Ausgestaltungen und Verbesserungen der Erfindung näher erläutert und beschrieben werden.

Es zeigt:
- Figur 1:: eine schematische Darstellung einer erfindungsgemäßen Gasmessanordnung
- Figur 2:: eine schematische Darstellung einer Prüfgasbereitstellungseinheit in einer ersten Ausführungsform, zur Verwendung in einer erfindungsgemäßen Gasmessanordnung,
- Figur 3:: eine schematische Darstellung einer Prüfgasbereitstellungseinheit in einer zweiten Ausführungsform, zur Verwendung in einer erfindungsgemäßen Gasmessanordnung.

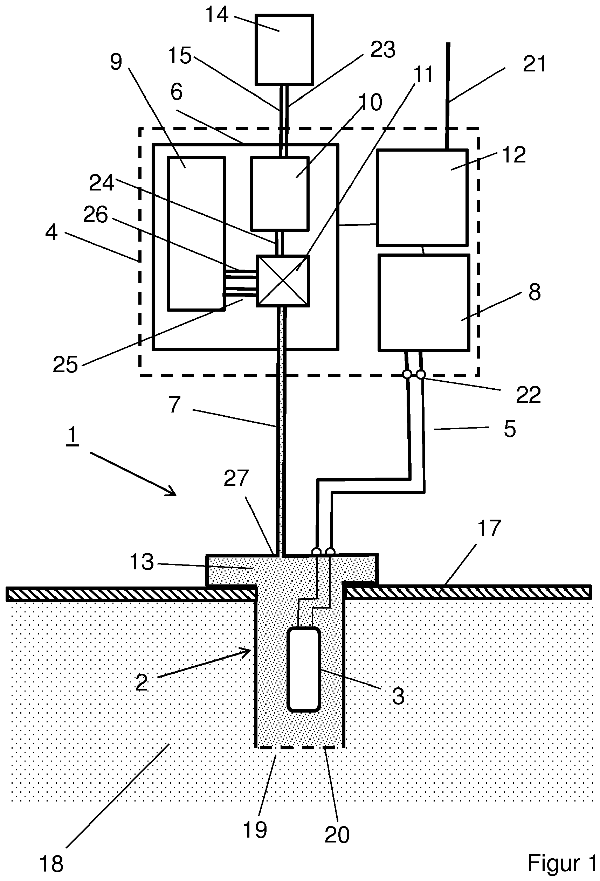

Es werde zunächst die Figur 1 betrachtet. Figur 1 zeigt schematisch und lediglich beispielhaft eine erfindungsgemäße Gasmessanordnung 1. Eine Gassensorvorrichtung 2, hier im Beispiel eine Einbauarmatur 2 für ein Gassensorelement 3, ist in die Rohrwand 17 eines Abgasrohres einer Feuerungsanlage eingebaut, wobei die Feuerungsanlage und weitere Einzelheiten der Abgasführung hier nicht dargestellt sind. Innen in der Einbauarmatur 2 befindet sich ein Gassensorelement 3, hier im Beispiel ein potentiometrisch arbeitender Zirkondioxid-Gassensor. Der Gassensor 3 hat eine Messelektrode, die ein erstes Messsignal in Abhängigkeit vom Vorhandensein und der Konzentration von oxidierbaren Gasbestandteilen, beispielsweise CO oder H₂ oder einem CO-Äquivalent COe, in der Gasatmosphäre nahe der Messelektrode erzeugt. Die Messelektrode wird daher auch als Messzone des Gassensors 3 bezeichnet. Weitere Einzelheiten des Sensors 3, wie weitere Elektroden, ein Heizelement, Einzelheiten der räumlichen Anordnung von Elektroden und Heizelement, Anbringung der Sensorsignalleitungen, eventuelle Zuführung einer Referenzgasatmosphäre und eines Prüfgases an eine Referenzelektrode des Sensorelementes 3, eventuell eine elektronische Signalvorverarbeitungseinheit an oder in der Einbauarmatur 2 oder auf dem Trägerteil des Gassensorelementes 3 selbst, oder andere, im Grunde bei Gassensoren bekannte Einzelheiten, sind hier in der Figur 1 nicht dargestellt.

Die Einbauarmatur 2 ist im Wesentlichen beispielsweise in etwa rohrförmig aufgebaut. An ihrer dem Gasraum 18 in dem Abgasrohr zugewandten Schmalseite 19 befinden sich Gaszuführungsöffnungen 20, damit Messgas aus dem Abgasrohr zu dem Sensorelement 3 gelangen kann. Die Einbauarmatur 13 ist in einer gasdicht ausgeführten Durchführung in der Rohrwand 17 mittels eines Einbauflansches befestigt und ragt in etwa senkrecht zu der Rohrwand 17 nach innen in das Abgasrohr.

Zu der Gasmessanordnung 1 gehört weiter ein Messgerät 4, welches hier im Beispiel getrennt von der Gassensorvorrichtung 2 angebracht ist. Zum Beispiel kann das Messgerät 4 an einer Wand angebracht sein, einige Meter von dem Abgasrohr der Feuerungsanlage entfernt. Der Abstand kann allerdings auch sehr kurz sein, so können die Gassensorvorrichtung 2 und das Messgerät 4 auch direkt aneinander liegend angeordnet sein. Die Gassensorvorrichtung 2 und das Messgerät 4 können auch in einem einzigen Gerät vereinigt sein.

Das Messgerät 4 kann ein kombiniertes Gerät sein, das sowohl die Messsignale von dem Gassensor 3 erfasst und auswertet, und zusätzlich unter Verwendung dieser Signale eine Sollwertvorgabe für die Brennersteuerung der Feuerungsanlage ermittelt und diese über eine Signalleitung 21 an die Brennerregelung der Feuerungsanlage (hier nicht dargestellt) übermittelt. Das Messgerät 4 kann auch ein Messwerterfassungs- und Übermittlungsgerät, ein Transmitter, sein ohne weitere Funktionen bezogen auf eine Brennerregelung oder Brennersteuerung.

Schließlich kann das Messgerät 4 auch ein für andere Anwendungen als die Feuerungsüberwachung vorgesehenes Gerät sein, beispielsweise ein Gerät zur Raumluftüberwachung oder zur Überwachung von verfahrenstechnischen Prozessen.

Das Messgerät 4 hat eine Messsignalverarbeitungs- und Auswertevorrichtung 8 und eine Steuervorrichtung 12. An einer Anschlussstelle 22 ist ein Signalanschlussmittel 5, ein Sensorsignalkabel 5 angeschlossen, das die Messsignale vom Gassensor 2 her übermittelt. Die Messsignale werden der Messsignalverarbeitungs- und Auswertevorrichtung 8 zugeführt, dort verarbeitet und ausgewertet. Die Messsignalverarbeitungs- und Auswertevorrichtung 8 ist signaltechnisch mit der Steuervorrichtung 12 verbunden. Somit ist die Steuervorrichtung 12 signaltechnisch über die Messsignalverarbeitungs- und Auswertevorrichtung 8 und das Sensorsignalkabel 5 signaltechnisch mit der Gassensorvorrichtung 2 verbunden.

In dem Messgerät 4 ist weiterhin eine Prüfgasbereitstellungseinheit 6 vorhanden. Diese hat eine Erzeugungseinheit 9 zur Erzeugung eines Sauerstoff-Teilstromes und eines Wasserstoff-Teilstromes. Weiterhin hat die Prüfgasbereitstellungseinheit 6 eine Trägergasbereitstellungseinheit 10, um einen Trägergasstrom bereitzustellen. Der Trägergasstrom ist hier im Beispiel ein Druckluftstrom. Die Druckluft dazu kommt aus einer anlagenseitig vorhandenen Druckluftversorgung 14 über eine Druckluftleitung 15, die an einer Druckluftanschlussstelle 23 an die Trägergasbereitstellungseinheit 10 angeschlossen ist. In der Trägergasbereitstellungseinheit 10 befindet sich eine Filtereinrichtung und eine Einrichtung, um den Druckluft-Volumenstrom einzustellen. Das kann im einfachsten Fall eine kritische Düse sein, oder ein Massendurchflussregler.

Die Trägergasbereitstellungseinheit 10 ist über eine interne Trägergasleitung 24 mit einer Prüfgaszusammensetzungseinheit 11 verbunden. Der Sauerstoff-Teilstrom wird der Prüfgaszusammensetzungseinheit 11 über eine Sauerstoff-Teilstromleitung 25, und der Wasserstoff-Teilstrom über eine Wasserstoff-Teilstromleitung 26 zugeführt.

Die Prüfgaszusammensetzungseinheit 11 arbeitet wie ein Mischer, in dem dem Trägergasstrom definierbare Anteile an Sauerstoff und/oder Wasserstoff zugemischt werden können. Selbstverständlich kann der Trägergasstrom auch ohne Sauerstoff und/oder Wasserstoffbeimischung durchgeleitet und an das Gassensorelement 3 herangeführt werden, beispielsweise um einen Offset-Abgleich durchzuführen.

Am Austritt der Prüfgaszusammensetzungseinheit 11 steht dann ein Prüfgas mit Luft als Trägergas und definierten Anteilen von Sauerstoff und/oder Wasserstoff zur Verfügung, oder auch nur Luft. Dieses wird über eine Prüfgasleitung 7 von dem Messgerät 4 an die Gassensorvorrichtung 2 geführt. Die Gassensorvorrichtung 2 hat dazu mindestens einen Prüfgasanschluss 27. Das Prüfgas verteilt sich im Inneren der Gassensorvorrichtung 2 und umspült dabei zumindest die Messzone des Gassensorelementes 3, wenn eine Referenzgaszone vorhanden ist, kann auch diese umspült werden, oder auch beide zusammen.

Die Prüfgasbereitstellungseinheit 6 ist signaltechnisch mit der Steuereinheit 12 verbunden. Die Steuereinheit 12 bestimmt die Zusammensetzung des für den jeweils anstehenden Funktionstest oder Kalibrierschritt erforderlichen Prüfgases, das dann in der Prüfgasbereitstellungseinheit 6 entsprechend bereitgestellt wird. Dabei kann die Steuereinheit 12 sowohl auf die Erzeugungseinheit 9, also auch auf die Trägergasbereitstellungseinheit 10 sowie auch auf die Prüfgaszusammensetzungseinheit 11 wirken. Sie bestimmt auch den Zeitpunkt und die Dauer der Prüfgasbeaufschlagung.

Hier im Beispiel ist die Erzeugungseinheit 9 eine Elektrolysezelle. Es kann eine Elektrolysezelle sein, die mit Wasser als Elektrolysemedium arbeitet und dazu einen kleinen Wasservorratsbehälter hat. Über die Elektrolysespannung und den Elektrolysestrom lässt sich die Menge an erzeugtem Sauerstoff und/oder Wasserstoff einstellen.

Zur definierten Einstellung und Mischung der Sauerstoff-, Wasserstoff- und Luftströme zwecks Erzeugung eines Prüfgases mit vorgegebenem Anteil an Wasserstoff und/oder Sauerstoff in der Luft können in der Mischeinheit 11 und/oder in den Trägergas-, Testgas- und Prüfgasleitungen 23, 25, 26, 24, 7 sogenannte kritische Düsen eingesetzt werden. Eine kritische Düse ist im Prinzip bekannt. Sie zeichnet sich dadurch aus, dass, solange ein kritisches Druckverhältnis, ein kritischer Druckabfall über der Düse aufrechterhalten oder überschritten ist, der Volumenstrom durch die kritische Düse hindurch konstant und weitgehend unabhängig von Druckschwankungen ist. Der kritische Druckabfall ist von geometrischen Düsenparametern bestimmt.

In einer vorteilhaften Ausführungsform kann eine kritische Düse, die den Volumenstrom des Prüfgases 13 einstellt, in oder nahe der Gassensorvorrichtung 2 mit dem beheizten Gassensorelement 3 angebracht sein. Die höhere Temperatur der Gassensorvorrichtung 2 würde dann auch die kritische Düse auf einer gegenüber der Umgebungs- und Messgastemperatur erhöhten höheren Temperatur belassen, wodurch sich Temperaturschwankungen der Umgebungs- und Messgastemperatur und/oder der Prüfgastemperatur weniger stark auf die kritische Düse auswirken würden. Dadurch lässt sich der Einfluss von Dichteschwankungen des Prüfgases 13, die durch möglicherweise im Prüfgas 13 oder der Umgebung der Prüfgasleitungen 7 auftretende Temperaturschwankungen bedingt sind, auf den Volumenstrom des Prüfgases 13 verringern. Die Aufrechterhaltung des kritischen Druckverhältnisses an den kritischen Düsen kann durch Beaufschlagen mit Überdruck oder durch die Erzeugung von Unterdruck erfolgen. Die Prüfgaszusammensetzungseinheit 11 kann in einer vorteilhaften Ausführungsform Pumpen auf der Saug- oder Zugseite der Testgas-, Trägergas- und/oder Prüfgasleitungen 23, 24, 25, 26, 7 einsetzen. Die Prüfgaszusammensetzungseinheit 11 kann allerdings auch mit Massenflussreglern, die nach einem thermischen und/oder elektropneumatischen oder anderen bekannten Prinzip arbeiten, zur definierten Einstellung der jeweiligen Volumenströme ausgestattet sein.

Die Steuervorrichtung 12 ist also signaltechnisch mit der Prüfgasbereitstellungseinheit 6 zum Umspülen des Gassensorelements 3 mit einem Prüfgas 13 vorgebbarer Zusammensetzung verbunden, und die Steuervorrichtung 12 ist signaltechnisch mit der Gassensorvorrichtung 2 zur Funktionsüberprüfung des Gassensorelements 3 unter Auswertung des von dem Gassensorelement 3 in Reaktion auf die Umspülung mit dem Prüfgas 13 erzeugten Sensorsignal verbunden.

Der Aufbau der Prüfgasbereitstellungseinheit 6 ist hier in der Figur 1 schematisch und exemplarisch in Funktionseinheiten unterteilt dargestellt. Eine oder mehrere Funktionseinheiten können dabei in einer gemeinsamen Baugruppe zusammengefasst und integriert sein.

In der Figur 1 ist die Gassensorvorrichtung 2 in die Rohrwand 17 einer Prozessleitung, hier im Beispiel eines Abgasrohres, eingebaut. Selbstverständlich erfordert es die erfindungsgemäße Gasmessanordnung 1 nicht, dass die Gassensorvorrichtung 2 in die Rohrwand einer Prozessleitung eingebaut ist.

Die erfindungsgemäße Gasmessanordnung hat in dem Messgerät eine Prüfgasbereitstellungseinheit 6 und eine Steuervorrichtung 12, und die Steuervorrichtung 12 ist signaltechnisch mit der Prüfgasbereitstellungseinheit 6 zum Umspülen des Gassensorelements 3 mit einem Prüfgas 13 vorgebbarer Zusammensetzung verbunden, und die Steuervorrichtung 12 ist signaltechnisch mit der Gassensorvorrichtung 2 zur Funktionsüberprüfung des Gassensorelements 3 unter Auswertung des von dem Gassensorelement 3 in Reaktion auf die Umspülung mit dem Prüfgas 13 erzeugten Sensorsignal verbunden, unabhängig davon, ob die Gassensorvorrichtung 2 in die Prozessleitung eingebaut ist oder nicht.

Der Aufbau der erfindungsgemäßen Gassensorvorrichtung erlaubt es beispielsweise, das Gassensorelement 3 im Stand-Alone-Betrieb, außerhalb der Verbrennungsanlage, aber bereits eingebaut in die Einbauarmatur 2, auf Funktion zu überprüfen oder sogar zu kalibrieren, ohne dass die Einbauarmatur 2 in einen Gasprüfstand eingebaut werden muss.

Selbstverständlich erlaubt es der Aufbau der erfindungsgemäßen Gassensorvorrichtung auch, das Gassensorelement 3 im eingebauten Zustand in einer Feuerungsanlage oder in einem sonstigen verfahrenstechnischen Prozess auf Funktion zu prüfen oder zu kalibrieren, ohne dass er dazu aus der Feuerungsanlage ausgebaut werden muss und ohne dass dazu die Feuerungsanlage oder der verfahrenstechnische Prozess gestoppt oder anderweitig in diesen eingegriffen werden muss.

Das Gassensorelement 3 kann zum Beispiel ein potentiometrischer Zirkondioxid-Gassensor sein mit einer Referenzelektrode auf einer Referenzseite und einer Messelektrode auf einer Messseite. Die Referenzseite und die Messseite sind dabei gastechnisch getrennt. Im üblichen Messeinsatz ist die Referenzseite mit einem Referenzgas konstanter und hoher Sauerstoffkonzentration beaufschlagt, meistens Luft mit 21% O₂. Zur Funktionsüberprüfung und Kalibrierung der Sauerstoff-Messfunktion des Gassensorelementes 3 kann es auch von Vorteil sein, die Referenzseite mit dem in der Prüfgasbereitstellungseinheit 6 des Messgerätes 4 bereitgestellten Prüfgas zu beaufschlagen, und die Messseite mit dem Referenzgas Umgebungsluft. Bei dieser Betriebsart der Funktionsüberprüfung oder Kalibrierung der Sauerstoff-Messfunktion des Gassensorelementes 3 besteht das Prüfgas vorteilhaft zu 100% aus Sauerstoff oder aus einer Mischung von Luft mit z.B. 50% O₂. In dieser Betriebsart der Funktionsüberprüfung beziehungsweise Kalibrierung wird dann die Referenzelektrode zur Messseite des Gassensorelementes 3 im Sinne der Erfindung. Die Referenzgasseite kann aufgrund des geringen Totraumvolumens mit einer sehr viel geringeren Prüfgasmenge beaufschlagt und die Erzeugungseinheit 9 samt Peripherie dadurch leistungs- und platzoptimiert aufgebaut werden.

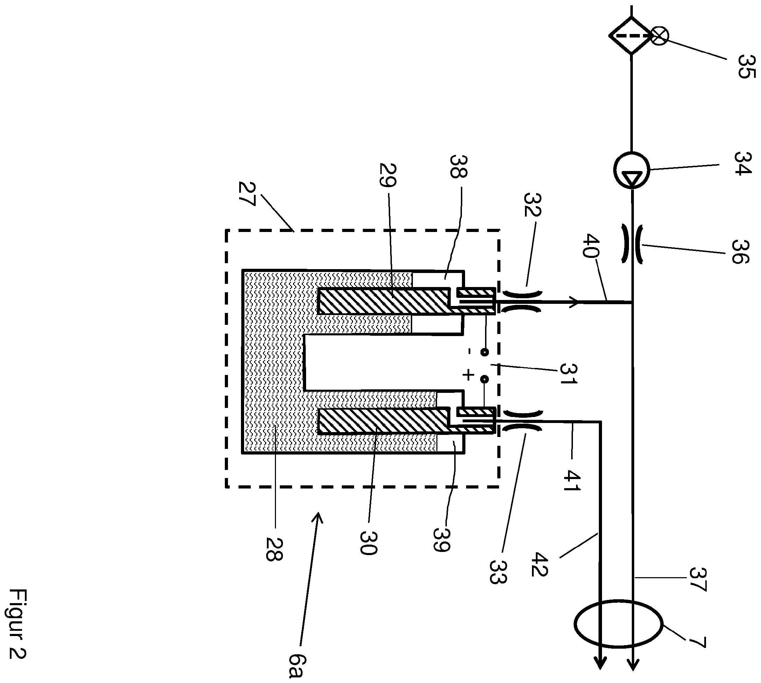

Es werde nun die Figur 2 betrachtet. Figur 2 zeigt schematisch eine Prüfgasbereitstellungseinheit 6a in einer ersten Ausführungsform, zur Verwendung in einer erfindungsgemäßen Gasmessanordnung. Die Prüfgasbereitstellungseinheit 6a gemäß Figur 2 ist dazu vorgesehen, um eine Funktionsüberprüfung des Gassensors im Stand-alone-Betrieb zu ermöglichen.

Die Prüfgasbereitstellungseinheit 6a hat einen Gasgenerator 27 zur Erzeugung von H₂ und O₂. Der Gasgenerator 27 ist ein Elektrolyseur, eine Elektrolysezelle, deren Funktion im Prinzip bekannt ist. Als Elektrolyt dient hier im Beispiel Wasser 28. Es gibt eine erste Elektrode 29, die Kathode, und eine zweite Elektrode 30, die Anode. Zwischen der ersten und zweiten Elektrode 29, 30 befindet sich eine Spannungsquelle 31. Der Minuspol ist mit der Kathode, der ersten Elektrode 29 verbunden, der Pluspol mit der Anode, der zweiten Elektrode 30. Bei Anlegen einer entsprechend geeignet gewählten Gleichspannung, einer sogenannten Zersetzungsspannung mit einem Wert von etwa größer 15 V, entsteht an der Kathode, der ersten Elektrode 29 H₂, und an der Anode, der zweiten Elektrode 30 entsteht O₂. Das H₂-Gas sammelt sich in einem ersten Gasraum 38 an der ersten Elektrode 29. Das O₂-Gas sammelt sich in einem zweiten Gasraum an der zweiten Elektrode 30.

Der erste Gasraum 38 ist über eine H₂-Leitung 40, in der eine zweite volumenstrombegrenzende Drossel 32 eingebaut ist, mit einer ersten Teilgasleitung 37 verbunden.

Der zweite Gasraum 39 ist über eine O₂-Leitung 41, in der eine dritte volumenstrombegrenzende Drossel 33 eingebaut ist, mit einer zweiten Teilgasleitung 42 verbunden.

Die erste und die zweite Teilgasleitungen 37, 42 sind Teil des Gaszuführungsmittels 7.

Mittels einer Pumpe 34, hier nur als Beispiel einer Überdruckpumpe, wird Umgebungsluft, gefiltert in einem Partikelfilter 35, über eine erste volumenstrombegrenzende Drossel 36 in der ersten Teilgasleitung 37 des Gaszuführungsmittels 7 zur Messgasseite des Gassensorelements geführt. Über den Bernoulli-Effekt wird das H₂-Gas aus dem ersten Gasraum 38 über die Drossel 32 gezogen und mit dem Umgebungsluftstrom mitgerissen und in der ersten Teilgasleitung 37 mit der Umgebungsluft vermischt. Das Luft/H₂-Gemisch wird über die erste Teilgasleitung 37 dem Gassensorelement zugeführt. Das O₂-Gas wird über die Drossel 33 und die zweite Teilgasleitung 42 ebenfalls dem Gassensorelement zugeführt.

Hier im Beispiel sei das Gassensorelement ein Festkörperelektrolyt-Gassensor mit einer Messgasseite und einer Referenzgasseite. Die Messgasseite hat eine COe-Elektrode, also eine Messelektrode, die eine Signalspannung erzeugt bei Vorhandensein von oxidierbaren Gasbestandteilen im Messgas. Die erste Teilgasleitung 37 führt hier das Luft/H₂-Gemisch der Messgasseite zu, und die zweite Teilgasleitung 42 führt das O₂-Gas der Referenzgasseite oder auch der Messgasseite zu.

Das Vorgehen bei einer Funktionskontrolle des Gassensorelementes ist die folgende. Zunächst findet eine Offset-Kalibrierung statt. Zur Offsetkalibrierung des Gassensors wird mittels der Pumpe 34 partikelgefilterte Umgebungsluft über die volumenstrombegrenzende Drossel 36 zur Messgasseite des Gassensorelementes geführt. Nach einer kurzen Zeit, beispielsweise 1-2 Minuten, bzw. wenn das Sensorsignal stabil ist, kann der Offset-Abgleich sowohl für die COe- als auch für die O₂-Seite durchgeführt werden. Damit ist die Offsetkalibrierung abgeschlossen.

Wenn der Gassensor mit der Einbauarmatur in einem Abgaskanal eingebaut ist, und wenn im Abgaskanal Unterdruck herrscht, kann auf die Pumpe 34 auch verzichtet werden.

Nach der Offset-Kalibrierung wird die Zersetzungsspannung an die Elektrolysezelle zwischen die beiden Elektroden 29, 30, zwischen die Kathode 29 und die Anode 30, angelegt. Über den Bernoulli-Effekt wird das H₂-Gas über die Drossel 32 gezogen und mit dem Umgebungsluftstrom mitgerissen und vermischt. Bereits geringste Konzentrationen in Höhe von beispielsweise 5-10ppm H₂ führen zu einem deutlichen Signalanstieg der COe-Seite und zu einem abspreizen des COe- vom O₂-Signal. Beide Reaktionen können zur Auswertung herangezogen werden. Damit ist die Funktion der COe-Seite nachgewiesen.

Nach einiger Zeit führt die Gasentwicklung in der Elektrolysezelle zu einem Druckanstieg, der - ohne zusätzlichen mechanischen Aufwand - das mit O₂-angereicherte Anodengas über die Drossel 33 an die Referenzgasseite des Gassensorelementes führt. Dies führt wiederum zu einem deutlichen Signalanstieg der O₂ (und der COe)-Seite. Damit ist die Funktion der O₂ (und der COe)- Seite nachgewiesen. In dem Fall, dass das mit O₂-angereicherte Anodengas auch an die Messgasseite des Gassensorelementes herangeführt wird, würde sich dann eine Abnahme des Signals einstellen, so dass auch damit die Funktion der O₂ (und der COe)- Seite nachgewiesen wäre.

Danach wird die Zersetzungsspannung am Elektrolyseur abgeschaltet. Nach einiger Zeit ist sowohl die Messgasseite als auch die Referenzgasseite freigespült. Die Anfangswerte für das COe und das O₂- Signal müssen dann wieder erreicht werden. Ist das der Fall, so ist das Abbruchkriterium für die Funktionskontrolle erfüllt, die Funktionsüberprüfung ist erfolgreich abgeschlossen.

Sollte das Abbruchkriterium - vor allem bedingt durch die langsamen Diffussionsvorgänge in den Leitungen der Anodenseite, also der O₂-Seite - nur langsam erreicht werden, so kann der Prozess durch ein Magnet-Absperrventil kurz vor dem Gassensor beschleunigt werden. Weiter kann durch amperometrische Verschaltung der Referenzelektrode als Pumpelektrode der Überschüssige O₂-Anteil schnell aus der Referenzkammer herausgepumpt werden. Bei diesem Vorgehen wären weitere interne Plausibilitätschecks zur Sensorfunktionsprüfung sinnvoll möglich.

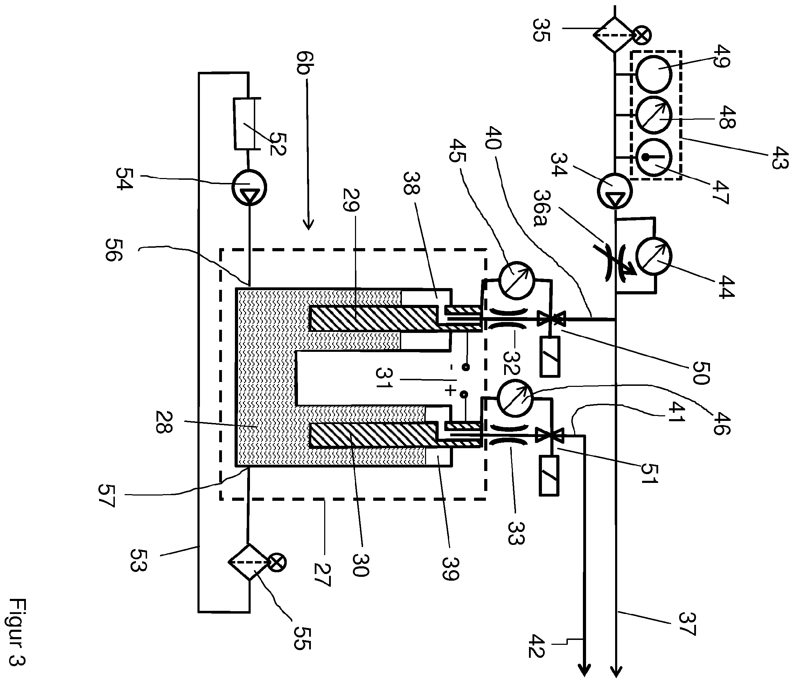

Es werde nun die Figur 3 betrachtet. Figur 3 zeigt schematisch eine Prüfgasbereitstellungseinheit 6b in einer zweiten Ausführungsform, zur Verwendung in einer erfindungsgemäßen Gasmessanordnung. Die Prüfgasbereitstellungseinheit 6b gemäß Figur 3 ist dazu vorgesehen, um eine Kalibrierung des Gassensors im Stand-alone-Betrieb zu ermöglichen. Dabei sind gleiche oder gleich wirkende Elemente oder Baugruppen in den Figuren mit denselben Bezugsziffern bezeichnet.

In der Ausführung nach Figur 3 wird über alle elektrisch verschließbaren Drosseln beziehungsweise Messblenden hinweg eine Differenzdruckmessung durchgeführt. Dazu ist über der ersten Drossel 36a ein erster Differenzdruckaufnehmer 44 geschaltet, über der zweiten Drossel 32 ein zweiter Differenzdruckaufnehmer 45, über der dritten drossel 33 ein dritter Differenzdruckaufnehmer 46. Über das Wirkdruckverfahren kann so der jeweilige Volumenstrom dV/dt abgeschätzt werden.

Zwischen dem Partikelfilter 35 und der Pumpe 34 befindet sich ein Umgebungsmodul 43 mit Messeinrichtungen zur Messung von Umgebungsparametern, nämlich hier im Beispiel einem Temperaturfühler 47 zur Erfassung der Umgebungstemperatur T_{amb}, einem Druckmesser 48 zur Erfassung des Luftdruckes p_{amb} in der Umgebung und einem Feuchtemesser 49 zur Erfassung der relativen Luftfeuchte in der Umgebung. Die Dichte erhält man durch das Umgebungsmodul 43 durch Messung von p_{amb} und T_{amb}.

Im Unterschied zu der Ausführungsform nach Figur 2 ist die erste Drossel als eine regelbare Drossel 36a zur Variation des Trägergastromes (dV/dt)_{Luft} ausgeführt.

Die zweite und dritte Drosseln 32, 33 sind als kritische Düsen ausgeführt, die konstante und bekannte Volumenströme (dV/dt)_{H2} und (dV/dt)_{O2} gewährleisten. Zusätzlich ist der zweiten Drossel 32 ein H₂ - Volumenstromregler 50 nachgeschaltet und der dritten Drossel 33 ist ein 02 - Volumenstromregler 51 nachgeschaltet.

Unter der Annahme, dass über die zweite Drossel 32 zunächst Luft und - nach einiger Zeit der Gasproduktion - über die zweite Drossel 32 reines H₂ bzw. über die dritte Drossel 33 reines O₂ strömen, können veränderliche und bekannte H₂-Konzentrationen in Luft an die Messelektrode des Sensors herangeführt und zur H₂-Mehrpunkt-Kalibrierung verwendet werden. Gleiches gilt für die O₂-Zweipunkt-Kalibrierung.

Sollte das kritische Druckverhältnis über der zweiten Drossel 32 oder der dritten Drossel 33 durch eine zu geringe Produktionsrate an Gasen nicht erreicht werden, so können zusätzliche Maßnahmen zur Druckerhöhung, zum Beispiel Kolben oder eine Druckmembran, vorgesehen werden. Alternativ könnte man auch mit nicht konstanten, aber bekannten Volumenströmen über der zweiten Drossel 32 oder der dritten Drossel 33 die Kalibriergaskonzentration abschätzen.

Sollte durch die Variation des Trägergaststromes über die erste Drossel 36a die Temperaturkonstanz des zu kalibrierenden Sensors nicht mehr eingehalten werden können, so kann die erste Drossel 36a auch nicht regelbar ausgeführt sein, wie in der Ausführungsform nach Figur 2. Zur Variation des H₂-Gehaltes im Kalibriergas kann dann alternativ die Produktionsrate an der Elektrolysezelle beispielsweise durch eine stromgeführte Spannungsbeaufschlagung verändert werden. Wenn dies reproduzierbar und langzeitstabil eingestellt ist, kann man bei annähernd konstantem Kalibriergasstrom kalibrieren.

Als Alternative zu einer H₂O-Elektrolysezelle 27 können zur Prüfgaserzeugung in der Prüfgasbereitstellungseinheit 6 auch andere Gasgeneratorprinzipien zur Erzeugung H₂-haltiger Gase verwendet, wie beispielsweise ein H₂-Testleck oder ein H₂-Generator auf Basis von Mikrosystemtechnik, sogenannte MEMS-Technologie.

In einer weiteren, nicht als Figur dargestellten, Ausführungsform können in der Prüfgasbereitstellungseinheit 6 zur definierten Erzeugung von H₂ und O₂ auch zwei Behälter, auch als Kartuschen bezeichnet, eingesetzt werden, in denen auf chemischem Wege H₂ bzw. O₂ erzeugt wird. In einer ersten Kartusche zur O₂-Erzeugung beispielsweise befindet sich eine erste Lösung, in der Sauerstoff chemisch gebunden ist, und ein Katalysator. Durch den Katalysator katalytisch unterstützt läuft eine chemische Reaktion ab, unter Bildung von O₂. Der Fachmann wird solche geeigneten Lösungen und Katalysatoren in der chemischen Fachliteratur finden können. Entsprechend befindet sich in einer zweiten Kartusche zur H₂-Erzeugung beispielsweise eine zweite Lösung, in der Wasserstoff chemisch gebunden ist, und ein Katalysator. Durch den Katalysator katalytisch unterstützt läuft eine chemische Reaktion ab, unter Bildung von H₂. Der Fachmann wird auch hier solche geeigneten Lösungen und Katalysatoren in der chemischen Fachliteratur finden können. Die Ausführungsform mit den beiden Kartuschen hat den Vorteil, dass sie wartungsfrei ist, stromlos abläuft, also keine Energieversorgung oder elektrische Regelung erfordert, und die Behältnisse oder Kartuschen bei Erschöpfung einfach ausgetauscht werden können, es ist Verbrauchsmaterial, gegebenenfalls könne sie recycelt oder wieder befüllt und erneut verwendet werden.

In der Ausführungsform nach Figur 3 ist auch schematisch und beispielhaft eine Befüllungsvorrichtung dargestellt, mit der der Wasservorrat 28 der Elektrolysezelle des Elektrolyseurs 27 automatisch nachgefüllt werden kann. Dazu ist Befüllungskreislauf eingerichtet, mit einer Befüllungsleitung 53, einem Vorratsgefäß 52, einer Befüllungspumpe 54 und einem Partikelfilter 55. Die Elektrolysezelle ist Teil des Befüllungskreislaufs, sie hat dazu mindestens einen Wasser-Zuleitungsanschluss 56 und mindestens einen Wasser-Abflussanschluss 57.

### Bezugszeichenliste

- 1: Gasmessanordnung
- 2: Gassensorvorrichtung, Einbauarmatur
- 3: Gassensorelement
- 4: Messgerät
- 5: Anschlussmittel
- 6: Prüfgasbereitstellungseinheit
- 6a: Prüfgasbereitstellungseinheit
- 6b: Prüfgasbereitstellungseinheit
- 7: Gaszuführungsmittel
- 8: Messsignalverarbeitungs- und Auswertevorrichtung
- 9: Erzeugungseinheit
- 10: Trägergasbereitstellungseinheit
- 11: Prüfgaszusammensetzungseinheit
- 12: Steuervorrichtung
- 13: Prüfgas
- 14: Druckluftversorgung
- 15: Druckluftleitung
- 16: Druckluftteilstrom
- 17: Rohrwand
- 18: Gasraum
- 19: Schmalseite
- 20: Gaszuführungsöffnungen
- 21: Signalleitung
- 22: Anschlussstelle
- 23: Druckluftanschlussstelle
- 24: interne Trägergasleitung
- 25: Sauerstoff-Teilstromleitung
- 26: Wasserstoff-Teilstromleitung
- 27: Gasgenerator, Elektrolyseur
- 28: Wasser
- 29: erste Elektrode, Kathode, H₂
- 30: zweite Elektrode, Anode, O₂
- 31: Spannungsquelle
- 32: zweite volumenstrombegrenzende Drossel
- 33: dritte volumenstrombegrenzende Drossel
- 34: Pumpe
- 35: Partikelfilter
- 36: erste Drossel
- 36a: erste Drossel, regelbar
- 37: erste Teilgasleitung
- 38: erster Gasstrom
- 39: zweiter Gasstrom
- 40: H₂-Leitung
- 41: O₂-Leitung
- 42: zweite Teilgasleitung
- 43: Umgebungsmodul
- 44: erster Differenzdruckaufnehmer
- 45: zweiter Differenzdruckaufnehmer
- 46: dritter Differenzdruckaufnehmer
- 47: Temperaturfühler
- 48: Druckmessgerät
- 49: Feuchtigkeitsfühler
- 50: H₂ - Volumenstromregler
- 51: O₂ - Volumenstromregler
- 52: Vorratsgefäß
- 53: Befüllungsleitung
- 54: Befüllungspumpe
- 55: Partikelfilter
- 56: Wasser-Zuleitungsanschluss
- 57: Wasser-Abflussanschluss

## Patentansprüche

1. Gasmessanordnung (1), welche umfasst
- eine Gassensorvorrichtung (2), die ein Gassensorelement (3) mit einer Messzone enthält,
- ein Messgerät (4), das eine Messsignalverarbeitungs- und Bereitstellungseinheit enthält, **dadurch gekennzeichnet, dass** das Messgerät (4) zusätzlich eine Prüfgasbereitstellungseinheit (6) zur Bildung eines Prüfgases (13) vorbestimmbarer und/oder veränderbarer Prüfgaszusammensetzung enthält, und dass die Gasmessanordnung (1) ein Gaszuführungsmittel (7) umfasst, um das Prüfgas (13) von der Prüfgasbereitstellungseinheit (6) in dem Messgerät (4) dem Gassensorelement (3) zuzuführen.

2. Gasmessanordnung (1) nach Anspruch 1, **dadurch gekennzeichnet, dass** die Prüfgasbereitstellungseinheit (6) eine Erzeugungseinheit (9) zur Erzeugung eines oder mehrerer Teilströme mit Gasbestandteilen des Prüfgases (13), eine Trägergasbereitstellungseinheit (10) zur Bereitstellung eines Trägergas-Teilstroms und eine Prüfgaszusammensetzungseinheit (11) zur Mischung des Trägergas-Teilstroms mit dem oder den in der Erzeugungseinheit (9) bereitgestellten Teilströmen der Gasbestandteile hat.

3. Gasmessanordnung (1) nach Anspruch 2, **dadurch gekennzeichnet, dass** die Prüfgasbereitstellungseinheit (6) eingerichtet ist, um einen Prüfgasstrom mit O₂ in vorgebbaren Konzentrationen oder einen Prüfgasstrom mit H₂ in vorgebbaren Konzentrationen, oder einen Prüfgasstrom mit O₂ und H₂ in vorgebbaren Konzentrationen bereitzustellen.

4. Gasmessanordnung (1) nach Anspruch 2, **dadurch gekennzeichnet, dass** die Trägergasbereitstellungseinheit (10) einen Anschluss an eine Druckluftversorgung (14) von außerhalb des Messgerätes (4) her hat, und dass die Trägergasbereitstellungseinheit (10) eine Vorrichtung zum Erzeugen eines Druckluft-Teilstromes (16) mit vorgebbarer Flussrate hat.

5. Gasmessanordnung (1) nach Anspruch 2, **dadurch gekennzeichnet, dass** die Trägergasbereitstellungseinheit (10) einen Kompressor oder einen Ventilator oder ein Gebläse zur Erzeugung eines Druckluft-Teilstromes (16) aus der Umgebungsluft hat.

6. Gasmessanordnung (1) nach Anspruch 2, **dadurch gekennzeichnet, dass** die Erzeugungseinheit (9) mit einer Elektrolysezelle versehen ist zur Erzeugung eines Sauerstoff-Teilstroms als Teilstrom eines ersten Gasbestandteils des Prüfgases (13) und zur Erzeugung eines Wasserstoff-Teilstromes als Teilstrom eines zweiten Gasbestandteiles des Prüfgases (13).

7. Gasmessanordnung (1) nach Anspruch 2, **dadurch gekennzeichnet, dass** die Erzeugungseinheit (9) mit wenigstens einem ersten und einem zweiten Behälter versehen ist, wobei in dem wenigsten ersten Behälter auf chemischem Wege O₂ erzeugt wird und wobei in dem wenigsten zweiten Behälter auf chemischem Wege H₂ erzeugt wird.

8. Gasmessanordnung (1) nach Anspruch 7, **dadurch gekennzeichnet, dass** in dem wenigstens ersten Behälter zur O₂-Erzeugung sich eine erste Lösung befindet, in der Sauerstoff chemisch gebunden ist, und ein Katalysator, wobei durch den Katalysator katalytisch unterstützt eine chemische Reaktion unter Bildung von O₂ abläuft, und dass in dem wenigstens zweiten Behälter zur H₂-Erzeugung sich eine zweite Lösung befindet, in der Wasserstoff chemisch gebunden ist, und ein Katalysator, wobei durch den Katalysator katalytisch unterstützt eine chemische Reaktion unter Bildung von H₂ abläuft.

9. Gasmessanordnung (1) nach Anspruch 2, **dadurch gekennzeichnet, dass** die Anordnung aus Erzeugungseinheit, Trägergasbereitstellungseinheit und Prüfgaszusammensetzungseinheit eine mit Wasser und Alkohol gefüllte Kartusche umfasst, über die ein Prüf-Luftstrom derart geführt wird, dass der Prüf-Luftstrom mit Alkoholdämpfen angereichert wird, so dass der mit Alkoholdämpfen angereicherte Prüf-Luftstrom das im Messgerät erzeugte Prüfgas darstellt.

10. Gasmessanordnung (1) nach Anspruch 2, **dadurch gekennzeichnet, dass** die Prüfgaszusammensetzungseinheit (11) eine Mischeinheit hat zur Mischung des Trägergas-Teilstroms mit dem oder den Teilströmen der Gasbestandteile.

11. Gasmessanordnung (1) nach einem der vorigen Ansprüche, **dadurch gekennzeichnet, dass** sich das Messgerät (4) getrennt von der Gassensorvorrichtung (2) befindet.

## Claims

1. Gas measuring arrangement (1) which comprises
- a gas sensor device (2) which contains a gas sensor element (3) with a measuring zone,
- a measuring device (4) which contains a measuring signal processing and provision unit,
**characterized in that** the measuring device (4) additionally contains a test gas preparation unit (6) for the formation of a test gas (13) of a pre-determinable and / or changeable test gas composition, and that the gas measuring arrangement (1) comprises a gas supply means (7) to supply the test gas (13) from the test gas supply unit (6) in the measuring device (4) to the gas sensor element (3).

2. Gas measuring arrangement (1) according to claim 1, **characterized in that** the test gas supply unit (6) has a generating unit (9) for generating one or more partial flows with gas components of the test gas (13), a carrier gas supply unit (10) for providing a carrier gas partial flow and a test gas composition unit (11) for mixing the carrier gas partial flow with the partial flow (s) of the gas components provided in the generating unit (9).

3. Gas measuring arrangement (1) according to claim 2, **characterized in that** the test gas supply unit (6) is set up to provide a test gas flow with O2 in predefinable concentrations or a test gas flow with H2 in predefinable concentrations, or a test gas flow with O2 and H2 in predefinable concentrations.

4. Gas measuring arrangement (1) according to claim 2, **characterized in that** the carrier gas supply unit (10) has a connection to a compressed air supply (14) from outside the measuring device (4), and that the carrier gas supply unit (10) has a device for generating a partial flow of compressed air (16) with a predeterminable flow rate.

5. Gas measuring arrangement (1) according to claim 2, **characterized in that** the carrier gas supply unit (10) has a compressor or a ventilator or a blower for generating a compressed air partial flow (16) from the ambient air.

6. Gas measuring arrangement (1) according to claim 2, **characterized in that** the generating unit (9) is provided with an electrolysis cell for generating a partial oxygen flow as a partial flow of a first gas component of the test gas (13) and for generation of a partial flow of hydrogen as a partial flow of a second gas component of the test gas (13).

7. Gas measuring arrangement (1) according to claim 2, **characterized in that** the generating unit (9) is provided with at least a first and a second container, wherein O2 is generated in the at least first container by chemical means and wherein H2 is chemically generated in the at least second container.

8. Gas measuring arrangement (1) according to claim 7, **characterized in that** in the at least first container for O2 generation there is a first solution in which oxygen is chemically bound, and a catalyst, with a chemical being supported by the catalyst catalytically reaction with the formation of O2 takes place, and that in the at least second container for H2 generation there is a second solution in which hydrogen is chemically bonded, and a catalyst, whereby a chemical reaction with formation is catalyzed by the catalyst of H2 expires.

9. Gas measuring arrangement (1) according to claim 2, **characterized in that** the arrangement of generating unit, carrier gas supply unit and test gas composition unit comprises a cartridge filled with water and alcohol, over which a test air flow is guided in such a way that the test air flow is enriched with alcohol vapors, so that the test air flow enriched with alcohol vapors represents the test gas generated in the measuring device.

10. Gas measuring arrangement (1) according to claim 2, **characterized in that** the test gas composition unit (11) has a mixing unit for mixing the carrier gas partial flow with the partial flow or flows of the gas constituents.

11. Gas measuring arrangement (1) according to one of the preceding claims, **characterized in that** the measuring device (4) is located separately from the gas sensor device (2).

## Revendications

1. Dispositif de mesure de gaz (1) qui comprend
- un dispositif capteur de gaz (2) qui contient un élément capteur de gaz (3) avec une zone de mesure,
- un dispositif de mesure (4) qui contient une unité de traitement et d'alimentation du signal de mesure,
**caractérisé en ce que** le dispositif de mesure (4) contient en plus une unité de préparation de gaz de test (6) pour la formation d'un gaz de test (13) de composition de gaz de test prédéterminable et / ou modifiable, et **en ce que** le dispositif de mesure de gaz (1) comprend un moyen d'alimentation en gaz (7) pour fournir le gaz de test (13) à partir de l'unité d'alimentation en gaz de test (6) dans le dispositif de mesure (4) à l'élément capteur de gaz (3).

2. Dispositif de mesure de gaz (1) selon la revendication 1, **caractérisé en ce que** l'unité d'alimentation en gaz de test (6) comporte une unité de génération (9) pour générer un ou plusieurs flux partiels avec des composants gazeux du gaz de test (13), un unité d'alimentation en gaz vecteur (10) pour fournir un flux partiel de gaz vecteur et une unité de composition de gaz de test (11) pour mélanger le flux partiel degaz vecteur avec le ou les flux partiels des composants de gaz prévus dans l'unité de génération (9).

3. Dispositif de mesure de gaz (1) selon la revendication 2, **caractérisé en ce que** l'unité d'alimentation en gaz de test (6) est agencée pour générer un flux de gaz de test avec O2 à des concentrations prédéfinies ou un flux de gaz de test avec H2 à des concentrations prédéfinies, ou un flux de gaz de test avec O2 et H2 à des concentrations prédéfinies.

4. Dispositif de mesure de gaz (1) selon la revendication 2, **caractérisé en ce que** l'unité d'alimentation en gaz vecteur (10) présente un raccordement à une alimentation en air comprimé (14) depuis l'extérieur du dispositif de mesure (4), et **en ce que** lunité d'alimentation en gaz vecteur (10) comprend un dispositif pour générer un flux partiel d'air comprimé (16) avec un débit prédéterminé.

5. Dispositif de mesure de gaz (1) selon la revendication 2, **caractérisé en ce que** l'unité d'alimentation en gaz vecteur (10) comporte un compresseur ou un ventilateur ou une soufflante pour générer un flux partiel d'air comprimé (16) à partir de l'air ambiant.

6. Dispositif de mesure de gaz (1) selon la revendication 2, **caractérisé en ce que** l'unité de génération (9) est pourvue d'une cellule d'électrolyse pour générer un flux partiel d'oxygène en tant que flux partiel d'un premier composant gazeux du gaz de test (13) et pour la génération d'un flux partiel d'hydrogène en tant que flux partiel d'un deuxième composant gazeux du gaz de test (13).

7. Dispositif de mesure de gaz (1) selon la revendication 2, **caractérisé en ce que** l'unité de génération (9) est munie d'au moins un premier et un deuxième conteneur, et que l'O2 est généré dans le moins premier conteneur par des moyens chimiques et quel dans le au moins deuxième conteneur est du H2 généré chimiquement.

8. Dispositif de mesure de gaz (1) selon la revendication 7, **caractérisé en ce que** dans l'au moins premier conteneur pour la génération d'O2, il y a une première solution dans laquelle l'oxygène est lié chimiquement, et un catalyseur, et que supporté par le catalyseur catalytiquement une réaction avec la formation d'O2 a lieu, et que dans le au moins deuxième récipient pour la génération de H2, il y a une deuxième solution dans laquelle l'hydrogène est chimiquement lié, et un catalyseur, par lequel une réaction chimique a lieu avec formation de H2 quie est catalysée par le catalyseur.

9. Dispositif de mesure de gaz (1) selon la revendication 2, **caractérisé en ce que** l'agencement d'unité de production, d'unité d'alimentation en gaz vecteur et d'unité de composition de gaz de test comprend une cartouche remplie d'eau et d'alcool, sur laquelle un flux d'air de test est guidé dans une telle manière dont le débit d'air d'essai est enrichi en vapeurs d'alcool, de sorte que le débit d'air d'essai enrichi en vapeurs d'alcool représente le gaz de test généré dans l'appareil de mesure.

10. Dispositif de mesure de gaz (1) selon la revendication 2, **caractérisé en ce que** l'unité de composition de gaz de test (11) comporte une unité de mélange pour mélanger le flux partiel de gaz vecteur avec le ou les flux partiels des constituants gazeux.

11. Dispositif de mesure de gaz (1) selon l'une des revendications précédentes, **caractérisé en ce que** le dispositif de mesure (4) est situé séparément du dispositif de détection de gaz (2).
